# EUROPEAN PATENT SPECIFICATION

(11) **EP 3 468 978 B1**
(45) Date of publication and mention of the grant of the patent: **25.03.2020**
(21) Application number: 16770098.8
(22) Date of filing: 14.06.2016
(51) Int. Cl.: A61K 47/64

(54) **HIGH-AFFINITY COMPOUND FOR TARGETED BORON NEUTRON CAPTURE THERAPY (T-BNCT) AND USE THEREOF**
HOCHAFFINE VERBINDUNG ZUR GEZIELTEN BORNEUTRONENFANGTHERAPIE (T-BNCT) UND VERWENDUNG DAVON
COMPOSÉ À HAUTE AFFINITÉ POUR THÉRAPIE CIBLÉE PAR CAPTURE DE NEUTRONS PAR LE BORE (T-BNCT) ET SON UTILISATION

(43) Date of publication of application: 17.04.2019
(73) Proprietor: Cassandro S.r.l., 70051 Barletta (Bari) (IT); Martellini, Maurizio, 20142 Milano (IT); Gherardi, Giuseppe, 40126 Bologna (IT)
(72) Inventor: MARTELLINI, Maurizio, I-20131 Milano (IT); GHERARDI, Giuseppe, I-40126 Bologna (IT)
(74) Representative: Modiano, Micaela Nadia
(86) International application number: PCT/IT2016/000155
(87) International publication number: WO 2017/216816

(56) References cited:
- EP-A1- 2 921 206
- Elisabetta Durisi et al: "Study of a DD compact neutron generator for BNCT.", internet article by instituto Nazionale de Fisica Nucleare, 18 April 2005 (2005-04-18), XP002767852, Retrieved from the Internet: URL:http://www.google.nl/url?sa=t&rct=j&q= &esrc=s&source=web&cd=7&cad=rja&uact=8&ved =0ahUKEwi2yr-84cTSAhXBoRQKHUKrCtEQFgg8MAY& url=http%3A%2F%2Fwww.slideserve.com%2Fluis a%2Fstudy-of-a-dd-compact-neutron-generato r-for-bnct&usg=AFQjCNFEDjUL8u6_BkVVu_Lsr4g tHd9_tw&sig2=BKnZD7tW6whgsNw17Wt3RQ [retrieved on 2017-03-07]
- POURYAVI MEHDI ET AL: "Radiation shielding design of BNCT treatment room for D-T neutron source", APPLIED RADIATION AND ISOTOPES, ELSEVIER, OXFORD, GB, vol. 99, 19 February 2015 (2015-02-19), pages 90-96, XP029583990, ISSN: 0969-8043, DOI: 10.1016/J.APRADISO.2015.02.016
- Y. KASESAZ ET AL.: "Optimization of the beam shaping assembly in the D-D neutron generators-based BNCT using the response matrix method", APPLIED RADIATION AND ISOTOPES, vol. 82, 2013, pages 55-59, XP002767853,
- SILVIO AIME ET AL: "Synthesis of Gd(III)-C-palmitamidomethyl-C'-DOTAMA-C6-o -carborane: a new dual agent for innovative MRI/BNCT applications", ORGANIC & BIOMOLECULAR CHEMISTRY, vol. 6, no. 23, 1 January 2008 (2008-01-01), page 4460, XP55336435, GB ISSN: 1477-0520, DOI: 10.1039/b808804g
- VERENA M. AHRENS ET AL: "Receptor-mediated uptake of boron-rich neuropeptide Y analogues for boron-neutron capture therapy", CHEMMEDCHEM, vol. 10, 2015, pages 164-172, XP002766078, WILEY - VCH VERLAG., WEINHEIM ISSN: 1860-7179 cited in the application
- PAOLO BOGGIO ET AL: "The hydroboration reaction as a key for a straightforward synthesis of new MRI-NCT agents", ORGANIC & BIOMOLECULAR CHEMISTRY, vol. 13, no. 11, 1 January 2015 (2015-01-01), pages 3288-3297, XP55336440, GB ISSN: 1477-0520, DOI: 10.1039/C4OB02291B

## Description

The present invention relates to a boron-containing compound which specifically locates at the tumor site. The invention also relates to such compound for use in tumor diagnosis and/or treatment. In addition, the invention is directed to an irradiation room for Boron Neutron Capture Therapy (BNCT) suitable to be set up in a hospital or in a clinic unit.

The "neutron capture therapy" (NCT) is a non-invasive therapeutic procedure for treating locally malignant tumors such as primary tumors arising from the central nervous system (such as brain tumors and spinal tumors) and recurrent head and neck cancers. It is a two step therapeutic procedure: first, a compound containing a non-radioactive isotope having a high propensity to capture slow neutrons is administered to the patient. The compound has no cytotoxic activity by itself and is designed so that it specifically localizes at the tumor site. Sometimes, the compound can also chelate a radionuclide (also referred to as "radioactive tracer", or "contrast agent"), like ⁶⁸Ga or ⁶⁴Cu: the presence of a chelated radionuclide is advantageous as it allows to display the biodistribution of the compound by means of an imaging technique (e.g. Positron Emission Tomography (PET) imaging). Since the most commonly used isotope is ¹⁰boron (¹⁰B), the procedure is generally known as "Boron Neutron Capture Therapy" (BNCT). In the second step, the tumor is irradiated with a thermal neutron beam; the neutrons are captured by the isotope nuclei of the compound previously delivered to and up-taken by the tumor cells and a nuclear fission reaction occurs, the products of which destroy the cell itself. If the isotope is ¹⁰B, the nuclear reaction releases high-energy ⁴He and ⁷Li ions which are biologically destructive for the tumor cells. Yet, the damage of the neutron capture and decay is restricted to a range smaller than 12 µm, which is the typical average dimension of a cell. ¹⁰B has more than 2000 times higher probability of neutron capture than the nuclides of the tissues.

BNCT is a very effective strategy for the treatment of tumors. Owing to the short treatment time which is needed and the low risk of treatment-related side effects, BNCT will likely become the radiotherapy of choice for specific groups of patients (Skold, 2010). In fact, doses up to 60-70 Gy can be delivered to the tumor cells in a single application, compared to the 30 applications (on a daily administration basis) in 6-7 weeks for conventional external beam photon irradiation.

Generally speaking, BNCT is currently successfully employed in treating tumors arising from the central nervous tissue such as glioma, malignant meningioma, cancers of the head and neck, and in treating melanomas. The use of BNCT in treating other tumors like, e.g. spinal cord tumors, soft tissue sarcoma of extremities, thyroid cancer, locally recurrent breast cancer and liver metastases, as well as in treating non-malignant diseases like, e.g. vascular restenosis after vascular angioplasty and rheumatoid arthritis (through a "radiation synovectomy") are currently under examination.

Presently, great efforts are made to make BNCT as targeted as possible, through boron-containing compounds which are able to localize specifically at the tumor site and to be up-taken at a high concentration by tumor cells, thus avoiding undesired side effects in normal tissues. Recently, the development of targeted BNCT (T-BNCT) has been focused on the most common malignant brain tumor: *glioblastoma multiforme* (GBM). GBM represents the most aggressive subgroup of malignant gliomas, with a median survival of 6 months following surgical resection alone and of about 14-17 months in patients who undergo the most aggressive combined treatment (US data, Barani, 2015). GBM shows a rapid tumor growth and wide-spreading invasion into the surrounding normal brain tissue (infiltrative growth pattern), thus resulting only partially removable by surgery. Studies have demonstrated that the external beam photon or particle based radio therapy fails to address the microscopic clusters of invading stem-like GBM cells (Sauerwein, 2012).

The purpose of targeted BNCT is to reach also these minor cells populations, thus improving the therapeutic effect well over the existing therapeutic protocols. The choice of GBM as the exemplary system for the development of T-BNCT is due to the fact that GBM shows a high level of chemo- and radioresistance, which is a primary cause for therapeutic failure in oncology. The success of T-BNCT in overcoming such resistance in GBM will likely imply its success in the treatment of many other tumor types.

The compounds developed for performing the above described therapy are sometimes referred to as "theranostic" compounds (or molecules, or drugs). The term "theranostic", which is a portmanteau joining "therapy" and "diagnostic", conveys the idea that the procedure can be at the same time both therapeutic and diagnostic: the nuclear reaction involving the boron isotopes grants a therapeutic effect killing the tumor cells, while diagnosis, precise localization of the tumor and visualization of the tumor modification after the treatment can be obtained through medical imaging systems, like PET.

Currently, BNCT can only be performed in nuclear reactors, a solution which requires complex installations and specialized personnel, resulting in a very expensive protocol, reduced availability and locations far from hospitals, which represent severe obstacles to the implementation of a complex clinical treatment planning. A nuclear reactor, in fact, is currently the only available facility equipped with a high-flux neutron beam capable of providing the mean number of neutron captures per tumor cell allowing for an effective treatment in a reasonable irradiation time. The high-flux neutron beam of a nuclear reactor is nowadays necessary to compensate the low up-take of the current commercial compounds for BNCT, such as boronophenylalanine(BPA) and mercapto-*closo*-undecahydrododecaborate ([B₁₂HₙSH]²⁻ 2Na⁺(BSH), when used *per se.*

In the light of the above, the main purpose of the present invention is to provide a new tumor-selective boron-comprising compound to be used in targeted BCNT and in imaging diagnosis. Within this purpose, a scope of the invention is to provide a compound which specifically binds with high affinity and high selectivity to receptors expressed at an increased degree by tumor cells rather than normal cells. Another scope of the invention is to provide a compound with an increased up-take by the targeted tumor cells, so that it effectively reaches the tumor intracellular environment. Another scope of the invention is to provide a compound which can be traced by imaging techniques like PET in order to evaluate its biodistribution within the body of the patient. A further scope of the invention is to provide a compound which is stable and soluble in the biological fluids and which is not cytotoxic by itself. Another scope of the invention is to provide an irradiation room for Boron Neutron Capture Therapy (BNCT) suitable to be set up in a hospital or in a clinic unit and which can be used in combination with an imaging equipment which exploits the presence of the radionuclide chelated to the compound, like a PET imaging equipment. Documents Org. Biomol. Chem. 2008,6,4460-4466 and Org. Biomol. Chem. 2015,13,3288-3297 both describe similar but different compounds for MRI/BNCT applications.

The above purpose and scopes, as well as further scopes which will be better clarified in the following, are reached, in a first aspect, by a compound having the following formula I: 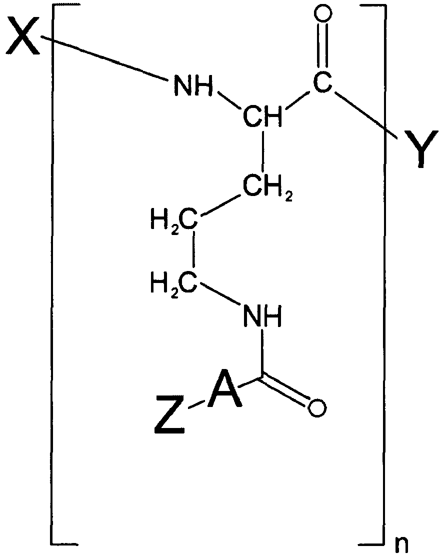 wherein:
X is a radionuclide chelating group;
Y is selected from the group consisting of a peptide;
A is selected from the group consisting of a bond, an alkylene group and a carboxyl group;
Z is selected from the group consisting of an optionally substituted carborane group and an optionally substituted dodecaborate group; n is an integer from 1 to 11.

The compound of formula I can be schematically indicated as comprising three moieties: the radionuclide chelating moiety (X-), the carborane- or dodecaborate-comprising moiety (-nCB-) and the peptide moiety (-Y).

Further scopes, features and advantages of the present invention will better appear from the detailed description provided in the following. In developing the compound, the first step was the selection of an appropriate moiety able to bind to the integrin receptors on the tumor cells surface. Maximizing the selectivity of the compound towards tumor cells rather than normal cells was a relevant issue to be taken into account. A high selectivity for tumor cells and a high capability to be up-taken within such cells leads to a remarkable concentration of the compound (and, ultimately, of the boron isotopes) within the tumor micro-environment. Peptides proved to be successful for the purpose, properly working as ligands to tumor cells receptors, in particular for tumors affecting the central nervous system, more particularly for *glioblastoma multiforme* (GBM). In a preferred embodiment of the present invention, the peptide can comprise the arginine-glycine-aspartic acid (RGD) sequence, which works as a ligand to the integrin receptors of the tumor cells. In another preferred embodiment, the peptide can be cyclic. Even more preferably, the peptide can be a cyclic peptide comprising the arginine-glycine-aspartic acid sequence (cRGD: cyclic arginyl-glycyl-aspartic acid). Other amino acids can be present in the cRGD, further to the arginine-glycine-aspartic acid sequence: as a non-limiting example, a suitable cRGD can be a cyclic structure consisting of phenylalanine, arginine, glycine, aspartic acid and lysine, linked to the carborane- or dodecaborate-comprising moiety through the lysine side chain.

The core of the compound is the boron-comprising structure; in order to convey a great number of boron atoms to the tumor micro-environment (thus increasing the boron concentration within the tumor cells), carborane and dodecaborate groups have been selected.

In the following therefore, and with reference to the compound of the invention, the expressions "boron-comprising moiety" and "carborane- or dodecaborate-comprising moiety" will be used interchangeably. As it is known in the art, carborane and dodecaborate are polyhedric boron-rich structures. In detail, a carborane is a cluster consisting of boron, carbon and hydrogen atoms: the most widely studied and employed carborane is the dicarborane with formula C₂B₁₀H₁₂; a dodecaborate (or *closo*-dodecaborate) is an ionic molecule with a symmetrical cluster of boron and hydrogen atoms with molecular formula B₁₂H₁₂²⁻. The use of carborane and dodecaborate in boron neutron captures therapy (BNCT) is currently under examination and continuous improvement. In Iguchi et al., Biomaterials, 56(2015), 10-17, a mercapto-*closo*-undecahydrododecaborate ([B₁₂HₙSH]²⁻ 2Na⁺, (BSH)), fused with an arginine tripeptide (acting as the tumor cells receptors ligand) was used. In Ahrens et al., Chem. Med. Chem., 10(2015), 164-172, 1 to 3 *closo-*dodecaborates were combined with a tumor-selective peptide (Modified Neuropeptide Y(NPY)).

In the present invention, the carborane- or dodecaborate-comprising moiety consists of 1 to 11 blocks, each block comprising an optionally substituted carborane group or an optionally substituted dodecaborate group (indicated as Z in formula I) and a spacer.

In a preferred embodiment, the carborane- or dodecaborate-comprising moiety can consist of 1 to 5 blocks. In a more preferred embodiment, the carborane- or dodecaborate-comprising moiety can consist of 3 blocks. The number of carborane or dodecaborate groups within the molecule must be evaluated with care in order to avoid cytotoxicity issues and grant the maximum solubility and stability of the whole compound.

As stated above, the carborane- or dodecaborate-comprising moiety Z is selected from an optionally substituted carborane group and an optionally substituted dodecaborate group. In a preferred embodiment, Z can be a closo-dodecaborate group; for example, Z can be the commercial mercapto-*closo-*undecahydrododecaborate ([B₁₂HₙSH]²⁻ 2Na⁺ (BSH)) group. In another preferred embodiment, Z can be a carborane group; more preferably, Z can be a dicarborane group, which is a carborane group with two carbon atoms, i.e. having formula -C₂B₁₀H₁₁. The dicarborane group can be linked to the A group either through one of the carbon atoms or through one of the boron atoms; preferably, the dicarborane group can be linked to the A group through a carbon atom.

The spacer is a linear chain with the purpose of placing the carborane or dodecaborate group at some distance from the radionuclide chelating group (X), from the peptide (Y) and, where n is greater than 1, from the other carboranes or dodecaborates present in the molecule. The spacer contains a group, indicated as A in formula I, which is selected from the group consisting of: a bond, an alkylene group and a carboxyl group. In an embodiment, the alkylene group and the carboxyl group can be linear. The choice of the A group is made considering the solubility of the resulting compound and the means for its synthesis process. In this regard, when A is an alkylene group or a carboxyl group, it can preferably have 1 to 5 carbon atoms. In addition, cytotoxicity of the resulting compound must also be taken into account: considering also the cytotoxicity issue, when A is an alkylene group or a carboxyl group, it can more preferably have 1 to 3 carbon atoms.

In view of the above, it follows that the A group is selected in order to optimize the solubility and stability properties of the resulting molecule while minimizing its cytotoxicity: in this regard, the selection of A is performed taking into account the number of carborane or dodecaborate groups, the specific chelating agent and the peptide/peptidomimetic present in the molecule.

As stated above, the binding of the compound (through its chelating moiety X) to a radionuclide ("radioactive tracer", or "contrast agent") allows to get a picture of the biodistribution of the compound within the body of the patient, which is of the utmost importance for both diagnostic and therapeutic purposes. In particular, the use of a radionuclide allows for a functional imaging of the molecule distribution, allowing the optimal timing for neutron irradiation. Such imaging may be obtained by, e.g., Positron Emission Tomography (PET). In addition, monitoring the biodistribution of the boron-containing compound allows optimizing the irradiation selectivity: a cytotoxic dose of the compound should reach tumor cells while a low to no cytotoxic dose of the compound should reach normal cells in the irradiation area. Radionuclides commonly used in PET imaging suit well also in this approach: as an example, ⁶⁸Ga or ⁶⁴Cu can be used. The preferred radionuclide used in clinical practice is ⁶⁸Ga, which is commonly available in every structure with PET diagnostic capability. ⁶⁸Ga allows the imaging of the compound biodistribution within a short time before neutron irradiation.

The choice of the radionuclide chelating moiety is very critical. In fact, the biodistribution of the compound can vary by a factor up to 20-30 from patient to patient and the chelating moiety must allow tracing both a high biodistribution and a low biodistribution. The preferred chelating groups are:
- 1,4,7,10-tetraazacyclododecane-1,4,7,10-tetraacetic acid (DOTA);
- 1,4,7-triazacyclononane-1-glutaric acid-4,7-diacetic acid (NODAGA); and
- 1,4,7,10-tetraazacyclododececane,1-(glutaric acid)-4,7,10-triacetic acid (DOTAGA).

DOTA, NODAGA and DOTAGA are more preferably used when a cRGD ligand is employed as the -Y moiety which binds to the integrin receptor.

It has been demonstrated that carboranes and dodecaborates are up-taken by the target tumor cells at a higher degree over other boron-containing molecules used in BNCT. In particular, the dodecaborates employed in Ahrens et al. showed a concentration within the target cells 30 times higher than control, and the dodecaborates employed in Iguchi et al. showed a concentration within the target cells 100 times higher than control, provided that BNCT by means of a nuclear reactor is performed. Such increased uptake may be explained supposing the presence of a mechanism comprising an integrin-mediated endocytosis (tumor cells overexpress integrins) and an enhanced permeability and retention (EPR) mechanism involving the blood vessels at the tumor site, which results in a longer residence time of the compound in the tumor micro-environment. In fact, such high up-take cannot actually be due to integrin-mediated endocytosis only: a density of 10⁷ integrin receptors per tumor cell (higher than the observed density) and/or an up-take rate higher than that measured would in fact be necessary. Instead, further mechanisms are likely to occur, such as a vesicle-mediated internalization, in view of the increased presence of vesicles (e.g. exosomes) in the tumor micro-environment than in normal tissues.

*In vivo* analyses demonstrated that the compound indicated as GPU-201 in Masunaga et al., World J.Oncol., 2012,3(3):103-112, which belongs to the cRGD-BSH family, remains in the bloodstream for a longer period than conventional boron-containing compounds; in addition, this compound also interacts with stem-like cells (which in Masunaga are referred to as "quiescent cells" (Qs) and are also known as "tumor initiating cells" (TIC)). It is reasonable to conclude that the interaction with stem-like cells occurs as a consequence of the long stay of the compound in the bloodstream rather than because of a common mechanism of vesicle-mediated up-take by both tumor and stem-like cells.

In view of the above, it is very likely that the radiotoxic effect of BNCT can affect also stem-like cells, thus helping to prevent the occurrence of relapses.

The compounds according to the present invention proved to provide even better results than known compounds comprising carborane o dodecaborate groups. Tests performed on the compounds of the invention demonstrated that they gather within the target tumor cells at a concentration at least 30 times higher than within control cells. In addition, the up-take of the compound of the invention is 20-100 times the value achievable with the compounds commonly used in BNCT treatment. Furthermore, the selectivity of the compounds of the invention for tumor cells is more than 5 times higher than normal cells.

These surprising results appear to be due to the choice of the various moieties and spacers which form the compound. In other words, a synergistic effect appears to arise as a consequence of the specific combinations of moieties and spacers of the compound of the invention.

It is noted that the structure as represented in formula I and schematically indicated as X-nCB-Y can result in a number of molecules according to the specific meanings of the X, Y, n, A and Z groups employed. Each resulting molecule will therefore be characterized by specific physical-chemical properties such as 3D geometry, steric hindrance, electric charge distribution, etc, resulting in different interactions in the tumor micro-environment, which in turn result, e.g., in a specific residence time in such micro-environment and a specific cellular up-take. The possibility to specifically select these features as the most appropriate according to, e.g. the type of tumor or the conditions of the patient allows a high degree of customization of the imaging process and/or of the treatment.

A preferred compound according to the invention is the compound having the following structure: 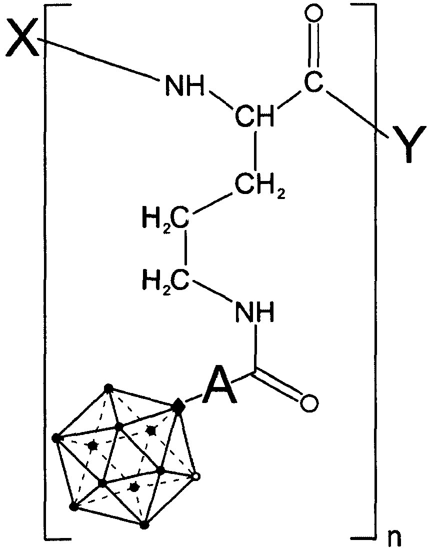 wherein X, Y, A and n are as disclosed above and further wherein:
• is ¹⁰BH;
∘ is CH;
◆ is C.

Another aspect of the invention is directed to a pharmaceutical composition which comprises a compound of formula I as described hereinabove. In one embodiment, such pharmaceutical composition can be in injectable form. In another embodiment, the pharmaceutical composition can further comprise one or more radionuclides (radioactive tracers). Examples of such radionuclides can be, e.g., ⁶⁸Ga or ⁶⁴Cu. Radionuclides are bound by the chelating group (X) of the compound of the invention, thus allowing the compound distribution at the tumor site to be traced by imaging (e.g. PET) techniques. Alternatively, when such imaging is desired, a radionuclide-containing formulation can be administered separately to the patient, at the same time of, or shortly before, or shortly after administration of the composition comprising the compound of formula I, so that chelation of the radionuclides by this compound occurs. In another preferred embodiment, the pharmaceutical composition can further comprise one or more pharmaceutical excipients, which are well-known to a person skilled in the field of pharmaceutical formulations and in particular of compositions for BNCT.

In still another aspect, the present invention is directed to the compound of formula I as described hereinabove for a medical use.

In a preferred embodiment, such medical use can be diagnosis. In particular, such use in diagnosis can be in combination with an imaging technique; preferably, the imaging technique can be PET (Positron Emission Tomography) imaging.

Preferably, such medical use can be in tumor diagnosis, more preferably in central nervous system tumors diagnosis, even more preferably in *glioblastoma multiforme* (GBM) diagnosis.

As a skilled person will immediately appreciate, the compound of formula I for use in diagnosis by imaging must bind a radionuclide ("radioactive tracer", or "contrast agent"), whose presence is necessary in order to display the biodistribution of the compound within the body of the patient.

In another preferred embodiment, such medical use can be the treatment of a disease. Preferably, such medical use can be the treatment of tumors, more preferably the treatment of central nervous system tumors, even more preferably the treatment of *glioblastoma multiforme* (GBM).

In addition, such medical use in the treatment of tumors can be in combination with the boron neutron capture therapy (BNCT).

From the description provided hereinabove, it follows that the compound of formula I according to the present invention can be advantageously used in both diagnosis and treatment of tumor at the same time. This possibility is granted by the presence in the molecule of both a radionuclide-chelating moiety and a carborane- or dodecaborate-comprising moiety. The radionuclide-chelating moiety allows for labelling and displaying the biodistribution of the compound at the tumor site within the body, while the carborane- or dodecaborate-comprising moiety, thanks to the presence of boron isotopes, which undergo a high energy-emitting nuclear reaction, allows the destruction of tumor cells.

The treatment can take place in an irradiation room, denominated "Theranostic Irradiation Room" (TIR), for boron neutron capture therapy (BNCT) comprising a neutron generator (NG) and a neutron beam shaping assembly (BSA), wherein the neutron generator (NG) is a compact deuterium-deuterium (D-D) plasma neutron generator.

The irradiation room can also comprise a medical imaging machine, such as a PET machine.

Said compact neutron generator complies well with purely therapeutic and/or diagnostic procedures in view of a series of advantages such as: small footprint, low installation and operational cost, operated by personnel with no need of authorization exceeding those of other radiological medical devices, no safety and security issues due to radioactive waste management issues, continuous availability, no radioactive waste, no risk of radioactive emissions, besides the obvious sheltering of the neutron generator to avoid the effects of the ionizing radiation against the hospital personnel and/or patients.

Said compact neutron generator has dimensions allowing its positioning in a hospital/clinic unit, so that the irradiation room can easily be set up directly in a hospital/clinic bunker unit. In addition to the neutron generator, the irradiation room can comprise auxiliary equipment for power supply and for cooling, and a command and monitoring console.

Advantageously the neutron generator is housed in an irradiation room designed to reduce the radiation exposure of workers and hospital staff to below the regulatory limits. The materials which are present in the irradiation room during the treatment with neutrons are selected in order to minimize the effect of fast neutrons and gamma rays produced by the compact neutron generator before the beam shaping assembly (BSA). The neutron generator can be operated by varying precisely the intensity and the duration of the neutron radiation based on patient and therapy requirements.

Advantageously the neutron beam shaping assembly is selectable from a set of neutron beam shaping assemblies based on patient and/or therapy requirements. Such neutron beam shaping assemblies may comprise components having different shapes, such as cylindrical or conical shapes, having different dimensions and made of different materials having different nuclear properties in order to direct the neutron beam at a given depth in the body organ and in order to precisely tailor the treatment based on the patient needs, that is within the so called "personalized therapy".

The irradiation room can be equipped with a series of beam shaping assemblies (BSA) with different geometries, each covering a range of possible patient requirements and/or biodistributions of the compound of formula I and/or tumor configurations.

Each treatment can advantageously use a beam shaping assembly geometry selected after a Monte Carlo calculation on a human phantom model tailored to the patient and therapy requirements. The beam shaping assembly can be remotely positioned in a 3D array varying the distance and the angle of irradiation according to the Monte Carlo calculation, since the maximum thermal flow depends on the tissue composition and on the tumor depth.

The 3D tumor image obtained for instance by Positron Emission Tomography (PET) or Magnetic Resonance Imaging (MRI) can be translated into the digital programming of the beam shaping assembly positioning and of the neutron beam modulation. The compact neutron generator can produce neutrons from a D-D reaction using a single ion-beam with acceleration voltage of 60-125 kV with a maximum thermal beam, at 15 cm from the neutron source, greater than 1x10⁷ thermal n/cm²/sec and preferably at least equal to 2x10⁷thermal n/cm²/sec.

A suitable compact neutron generator can be for instance the model DD110M developed by Adelphi Technology Inc., or also the upgraded model DD110MBwhich uses 4 ion beams to produce 5x10⁷ thermal n/cm²/sec. The thermal neutron beam spot size on the DD-110M is around 16 cm², while the thermal neutron beam spot size on the DD-110MB can be further reduced, and the beam increased, by using a conical collimator allowing a more tumor-focused BNCT (Bergaoui et al. "Development of a new deuterium-deuterium (D-D) neutron generator for prompt gamma-ray neutron activation analysis, Applied Radiation and Isotopes" Applied Radiation and Isotopes Volume 94, December 2014, Pages 319-327).

Advantageously, the DD110M and the DD110MB thermal neutron generators can be operated at a higher voltage so as to be suitable for T-BNCT at the biodistribution and boron up-take levels achievable by the compound of the present invention.

Furthermore, as shown in figures 1 and 2, the neutron generator (NG) and the beam shaping assembly (BSA) can be swiveled around the target area within an organ (TA) in order to direct the neutron beam (NB) towards the target area (TA) in a number of different directions.
Figure 1 shows the swiveling of the neutron generator and of the beam shaping assembly in a lateral view.
Figure 2 shows the swiveling of the neutron generator and of the beam shaping assembly in a view from the above. Figure 2 also shows, within dotted lines, an example of the area of displacement (DA) of the neutron generator and of the beam shaping assembly. In view of the above, it has been established that the present invention fully accomplishes the purpose and the scopes put forward hereinabove.

In fact, the selection of the particular moieties of the compound of formula I improves the selectivity of the compound for tumor cells over normal cells; also the compound uptake by tumor cells is increased over normal cells, thus resulting in a greater concentration of the compound within tumor cells than within normal cells. It follows that, advantageously, the compound of the invention leads to maximize the number of tumor cells which are killed by the destructive nuclear reaction involving the boron isotopes, while minimizing the number of normal cells killed. This leads to a patient-tailored, targeted therapy which allows preserving the healthy tissue.

Further, the invention allows the T-BNCT to be performed by the medical personnel commonly working in nuclear medicine departments and by means of a smaller, more comfortable equipment: these features will increase the accessibility of patients to T-BNCT thus allowing a greater number of patients to be treated.

Furthermore, the boron compound characterization and the thermal neutron beam generated by a compact neutron generator will be optimized accordingly to the patient and/or therapy requirements.

The economic advantage to realize a hospital irradiation room for T-BNCT through a compact thermal neutron generator via the D-D reaction including a PET imaging equipment aimed to the pharmacokinetics of the administrated highly affine boron compound lies in:
1) the very modest capital cost of this generator with respect to the one associated, for instance, to a middle energy (25-30 MeV) proton - cyclotron and a beryllium target (such as the Sumitomo Heavy Industries Ltd HM-30 machine, which weighs 60 tons and must be placed in a highly shielded bunker of at least 18m x 18m area);
2) the very limited operational costs, including the costs of the non-medical staff employed and maintenance;
3) the almost absence of radioactive waste management costs, besides the costs due to the re-placement - in general, roughly, after several thousands hours - of the titanium target used by the Adelphi-like compact neutron generators; and
4) in the possibility to treat non-stop several patients daily without a continuous recalibration of the thermal neutron beam generated by a proton - cyclotron.

All the features disclosed in the present application with reference to a certain aspect of the invention must be considered to regard as well also the other aspects of the inventions, where applicable, even if they have not been explicitly repeated.
In memory of Marina Gherardi

## Claims

1. A compound of Formula I: 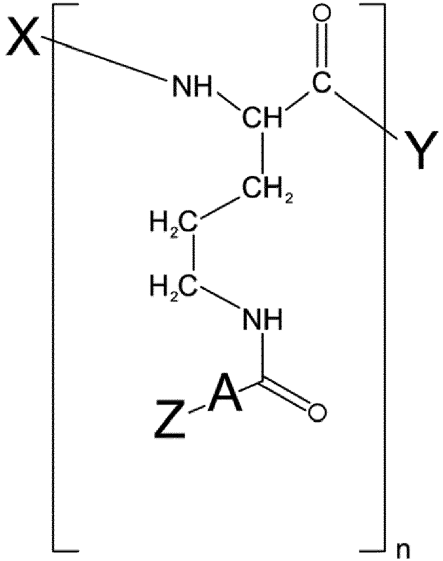 wherein:
X is a radionuclide chelating group;
Y is a peptide;
A is selected from the group consisting of a bond, an alkylene group and a carboxyl group;
Z is selected from the group consisting of an optionally substituted carborane group and an optionally substituted dodecaborate group;
n is an integer from 1 to 11.

2. The compound according to claim 1 wherein Z is a closo-dodecaborate group.

3. The compound according to claim 1 wherein Z is a dicarborane group.

4. The compound according to any of claims 1 to 3 wherein A is selected from the group consisting of an alkylene group and a carboxyl group having from 1 to 3 carbon atoms.

5. The compound according to any of claims 1 to 4 wherein n is 3.

6. The compound according to any of claims 1 to 5 wherein X is selected from the group consisting of:
(i) 1,4,7, 1 0-tetraazacyclododecane-1,4,7,10-tetraacetic acid (DOTA);
(ii) 1,4,7-triazacyclononane-1-glutaric acid-4,7-diacetic acid (NODAGA); and
(iii) 1,4,7,10-tetraazacyclododececane,1-(glutaric acid)-4,7,10-triacetic acid (DOTAGA);

7. The compound according to any of claims 1 to 6 wherein Y is a peptide comprising the arginine-glycine-aspartic acid (RGD) amino acid sequence.

8. The compound according to any of claims 1 to 7 having formula: 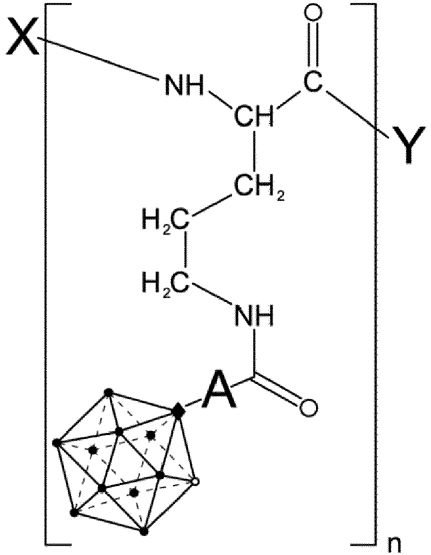 wherein X, Y, A and n are as defined in claims 1-7 and further wherein:
• is ¹⁰BH;
∘ is CH;
◆ is C.

9. The compound according to any of claims 1-8 for a use selected from the group consisting of diagnosing a tumor, treating a tumor and diagnosing and treating at the same time a tumor in a patient in need thereof by administering a pharmaceutically effective amount of said compound to said patient.

10. The compound for use according to claim 9, in combination with the boron neutron capture therapy (BNCT).

11. The compound for use according to claim 9 or claim 10, in combination with an imaging technique, preferably Positron Emission Tomography (PET) imaging.

12. The compound for use according to any of claims 9-11 wherein said tumor affects the central nervous system.

13. The compound for use according to claim 12 wherein the tumor is *glioblastoma multiforme* (GBM).

14. A pharmaceutical composition comprising a compound of formula I according to any of claims 1-8.

## Patentansprüche

1. Eine Verbindung der Formel I 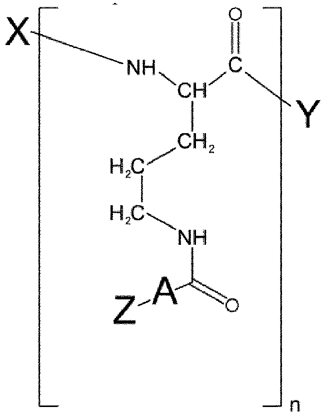 worin
X eine Radionuklid- chelatbildende Gruppe ist;
Y ist ein Peptid;
A ist gewählt aus der Gruppe bestehend aus einer Bindung, einer Alkylengruppe und einer Carboxylgruppe;
Z ist gewählt aus der Gruppe bestehend aus einer wahlweise substituierten Carborangruppe und einer wahlweise substituierten Dodecaboratgruppe;
n ist eine ganze Zahl von 1 bis 11.

2. Die Verbindung gemäß Anspruch 1, worin Z eine closo-Dodecaboratgruppe ist.

3. Die Verbindung gemäß Anspruch 1, worin Z eine Dicarborangruppe ist.

4. Die Verbindung gemäß irgendeinem der Ansprüche 1 bis 3, worin A gewählt ist aus der Gruppe bestehend aus einer Alkylengruppe und einer Carboxylgruppe mit 1 bis 3 Kohlenstoffatomen.

5. Die Verbindung gemäß irgendeinem der Ansprüche 1 bis 4, worin n 3 ist.

6. Die Verbindung gemäß irgendeinem der Ansprüche 1 bis 5, worin X gewählt ist aus der Gruppe bestehend aus:
(i) 1,4,7,10-Tetraazacyclododecan-1,4,7,10-tetraessigsäure (DOTA);
(ii) 1,4,7-Triazacyclononan-1-glutarsäure-4,7-diessigsäure (NODAGA); und
(iii) 1,4,7,10-Tetraazacyclododecan,1-(glutarsäure)-4,7,10-triessigsäure (DOTAGA).

7. Die Verbindung gemäß irgendeinem der Ansprüche 1 bis 6, worin Y ein Peptid ist, das die Arginin-Glycin-Asparaginsäure (RGD) Aminosäuresequenz umfasst.

8. Die Verbindung gemäß irgendeinem der Ansprüche 1 bis 7, die folgende Formel hat: 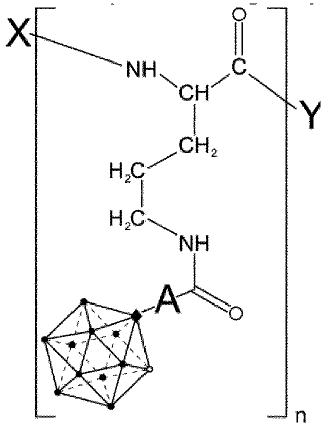 worin X, Y, A und n wie in Ansprüchen 1-7 definiert sind, und weiter worin:
• ¹⁰BH ist;
∘ CH ist;
◆ C ist.

9. Die Verbindung gemäß irgendeinem der Ansprüche 1-8 für eine Verwendung gewählt aus der Gruppe bestehend aus Diagnostizieren eines Tumors, Behandeln eines Tumors und gleichzeitigem Diagnostizieren und Behandeln eines Tumors in einem Patienten, der dies benötigt, durch Verabreichen einer pharmazeutisch wirksamen Menge der genannten Verbindung an den genannten Patienten.

10. Die Verbindung für die Verwendung gemäß Anspruch 9, in Kombination mit der Bor-Neutroneneinfangtherapie (BNCT).

11. Die Verbindung für die Verwendung gemäß Anspruch 9 oder Anspruch 10, in Kombination mit einem bildgebenden Verfahren, vorzugsweise der Positronen-Emissions-Tomographie(PET)-Bildgebung.

12. Die Verbindung für die Verwendung gemäß irgendeinem der Ansprüche 9-11, worin der genannte Tumor das Zentralnervensystem betrifft.

13. Die Verbindung für die Verwendung gemäß Anspruch 12, worin der Tumor *Glioblastoma multiforme* (GBM) ist.

14. Eine pharmazeutische Zusammensetzung umfassend eine Verbindung der Formel I gemäß irgendeinem der Ansprüche 1-8.

## Revendications

1. Composé de formule I : 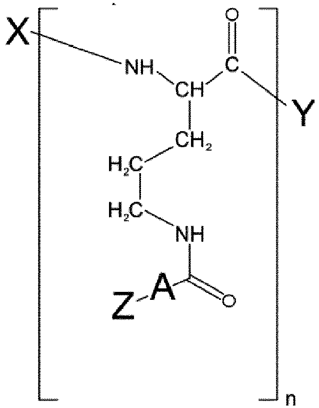 dans laquelle :
X est un groupe chélateur de radionucléide ;
Y est un peptide ;
A est choisi dans l'ensemble constitué par une liaison, un groupe alkylène et un groupe carboxy ;
Z est choisi dans l'ensemble constitué par un groupe carborane en option substitué et un groupe dodécaborate en option substitué ;
n est un nombre entier valant de 1 à 11.

2. Composé selon la revendication 1, dans lequel Z est un groupe closo-dodécaborate.

3. Composé selon la revendication 1, dans lequel Z est un groupe dicarborane.

4. Composé selon l'une quelconque des revendications 1 à 3, dans lequel A est choisi dans l'ensemble constitué par un groupe alkylène et un groupe carboxy ayant de 1 à 3 atomes de carbone.

5. Composé selon l'une quelconque des revendications 1 à 4, dans lequel n est égal à 3.

6. Composé selon l'une quelconque des revendications 1 à 5, dans lequel X est choisi dans l'ensemble constitué par :
(i) l'acide 1,4,7,10-tétraazacyclododécane-1,4,7,10-tétraacétique (DOTA) ;
(ii) le 1,4,7-triazacyclononane-1-acide glutarique-acide 4,7-diacétique (NODAGA) ; et
(iii) le 1,4,7,10-tétraazacyclododécécane,1-(acide glutarique)-acide 4,7,10-triacétique (DOTAGA).

7. Composé selon l'une quelconque des revendications 1 à 6, dans lequel Y est un peptide comprenant la séquence d'acides aminés arginine-glycine-acide aspartique (RGD).

8. Composé selon l'une quelconque des revendications 1 à 7, ayant la formule : 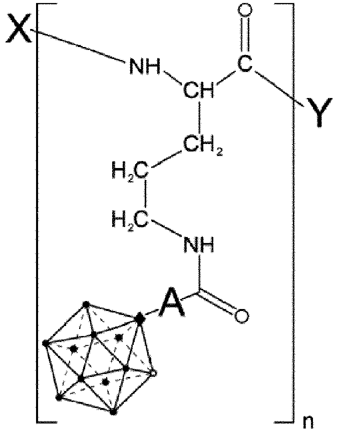 dans laquelle X, Y, A et n sont tels que définis dans les revendications 1 à 7 et en outre dans laquelle :
• est ¹⁰BH ;
∘ est un groupe CH ;
• est un atome de carbone.

9. Composé selon l'une quelconque des revendications 1 à 8, destiné à une utilisation choisie dans l'ensemble constitué par le diagnostic d'une tumeur, le traitement d'une tumeur et le diagnostic et le traitement en même temps d'une tumeur chez un patient en ayant besoin, par administration d'une quantité pharmaceutiquement efficace dudit composé audit patient.

10. Composé destiné à une utilisation selon la revendication 9, en association avec la thérapie par capture de neutrons par le bore (BNCT).

11. Composé destiné à une utilisation selon la revendication 9 ou la revendication 10, en association avec une technique d'imagerie, de préférence l'imagerie par tomographie par émission de positons (PET).

12. Composé destiné à une utilisation selon l'une quelconque des revendications 9 à 11, dans lequel ladite tumeur affecte le système nerveux central.

13. Composé destiné à une utilisation selon la revendication 12, dans lequel la tumeur est un glioblastome multiforme (GBM).

14. Composition pharmaceutique comprenant un composé de formule I selon l'une quelconque des revendications 1 à 8.
